# EUROPÄISCHE PATENTSCHRIFT

(11) **EP 3 243 428 B1**
(45) Veröffentlichungstag und Bekanntmachung des Hinweises auf die Patenterteilung: **26.08.2020**
(21) Anmeldenummer: 17178300.4
(22) Anmeldetag: 04.02.2010
(51) Int. Cl.: A61B 3/10, A61B 3/11, A61B 3/113

(54) **VERFAHREN UND VORRICHTUNG ZUR VERORDNUNG EINES BRILLENGLASES**
METHOD AND DEVICE FOR PRESCRIBING A SPECTACLE LENS
PROCÉDÉ ET DISPOSITIF DE PRESCRIPTION DE VERRE DE LUNETTES

(30) Priorität: 26.02.2009 DE 102009010467
(43) Veröffentlichungstag der Anmeldung: 15.11.2017
(62) Teilanmeldung aus: 12002380.9
(73) Patentinhaber: Carl Zeiss Vision GmbH, 73430 Aalen (DE)
(72) Erfinder: Kratzer, Timo, 73434 Aalen (DE); Cabeza-Guillén, Jesús-Miguel, 73434 Aalen (DE); Kelch, Gerhard, 73434 Aalen (DE)
(74) Vertreter: Glawe, Delfs, Moll

(56) Entgegenhaltungen:
- DE-A1- 19 950 790
- DE-A1-102005 003 699
- US-A1- 2003 223 037
- US-A1- 2007 025 642

## Beschreibung

Die Erfindung bezieht sich auf ein Verfahren zur Verordnung eines Brillenglases, bei dem mindestens zwei Parameter eines Systems eine Referenzstruktur aufweisendes Auge/Brille bestimmt werden und bei dem für die Bestimmung des jeweiligen Parameters das Auge jeweils mittels eines separaten Messgerätes photographisch aufgenommen wird.

Die Erfindung betrifft ferner eine Vorrichtung zum Durchführen des vorstehend genannten Verfahrens.

Es ist bekannt, zum Optimieren von Brillengläsern, insbesondere von individuellen Gleitsichtgläsern, verschiedene Parameter des Systems Auge/Brille zu berücksichtigen. Diese Parameter sind beispielsweise der Pupillenabstand PD, der Hornhaut-Scheitelabstand HSA, der Vorneigungswinkel α des Brillenglases, die Fassungsscheibenwinkel α_{R}, α_{L} für das rechte und linke Brillenglas sowie die Lage des optischen oder mechanischen Augendrehpunkts Z', M. Unter individuellen Gleitsichtgläsern versteht man Gleitsichtgläser, bei denen mindestens ein individueller Gebrauchsparameter bei der Berechnung des Brillenglases berücksichtigt wird und die mittels Freiformtechnologie gefertigt werden, wie dies z.B. in der Deutschen Optikerzeitung DOZ 4 + 5/2000 von W. Grimm und G. Kelch in dem Aufsatz "Grada® Individual: Konzeption, Fertigung und Anpassung", in der EP 857 993 B1 und/oder der EP 562 336 B1 beschrieben ist.

Bei nicht-individuellen Brillengläsern werden diese Parameter aus statistischen Mittelwerten eines repräsentativen Bevölkerungsquerschnitts ermittelt. Bei individuellen Brillengläsern hingegen werden die Parameter individuell an dem jeweiligen Brillenträger gemessen, beispielsweise mit so genannten Videozentriersystemen, wie sie von der Anmelderin unter den Typenbezeichnungen "i.Terminal" und "Relaxed Vision Terminal" hergestellt und vertrieben werden. Auch die DE 10 2005 003 699 A1 beschreibt ein derartiges Videozentriersystem. Diese Videozentriersysteme sind jedoch bisher nicht in der Lage, eine Optimierung des Brillenglases mit Hilfe der Augendrehpunktlage in einer ausreichend exakten Weise zu ermöglichen.

Wenn man die Augendrehpunktlage berücksichtigen möchte, kann man diese in bekannter Weise aus der Augenlänge über die mittlere Sphäre des verordneten Brillenglases ableiten. Dabei werden allerdings viele Annahmen gemacht, die mit den tatsächlichen Gegebenheiten nicht ausreichend übereinstimmen.

Der Zusammenhang zwischen Augenlänge und der Sphäre des verordneten Brillenglases wird oft vereinfacht als linear angenommen. Dies ist aber in der Realität nicht der Fall, weil sowohl die Krümmung der Hornhaut als auch die Augenlinse sowie die Augenlänge teilweise sehr unabhängig voneinander wachsen bzw. sich unterschiedlich entwickeln.

Im Allgemeinen ist es ausreichend, den Augendrehpunkt als punktförmiges Drehzentrum innerhalb des Auges zu betrachten. Die Erfindung umfasst jedoch ganz allgemein auch ein ausgedehntes, im Allgemeinen näherungsweise kugelförmiges Augendrehpunktsgebiet. Zur Beschreibung der Erfindung und des Standes der Technik wird nachfolgend der Einfachheit halber von einem punktförmigen Augendrehpunkt ausgegangen, soweit nichts Anderes erwähnt ist.

Üblicherweise geht man bei der Verordnung einer Brille so vor, dass die Brillengläser auf der Grundlage einer subjektiven Refraktion sowie einer Videozentriermessung verordnet und zentriert werden.

Dabei ist von Nachteil, dass es keine Referenzierung zwischen der Refraktionsbestimmung und der Zentrierung gibt, weil beide Vorgänge mit unterschiedlichen Apparaturen durchgeführt werden. Wenn die Refraktion beispielsweise mit einem Phoropter ermittelt wird, kann es vorkommen, dass der Phoropter um einen ersten Winkel relativ zu der der Linie verkippt ist, die die beiden Pupillenmittelpunkte des Auges verbindet. Ferner kann es vorkommen, dass die gewählte Brillenfassung relativ zu dieser Linie um einen zweiten Winkel verdreht ist. Im ungünstigsten Falle können sich die beiden Winkel addieren, was zu einer Diskrepanz von mehreren Winkelgraden in den Achsen der Zylinder zwischen der Verordnung und der ausgeführten Korrektur der fertigen Brille führen kann.

Aus der WO 2006/106248 A1 sind ein Verfahren und eine Vorrichtung zum Bestimmen der Augendrehpunktlage bekannt. Bei diesem bekannten Verfahren blickt ein Proband in ein fernrohrartiges Gerät. Die Ausrichtung des Gerätes im Raum wird durch einen am Gerät befindlichen, dreidimensional wirksamen Sensor bestimmt. Der Proband trägt an seinem Kopf einen weiteren derartigen Sensor, der die Lage und Ausrichtung seines Kopfes bestimmt. In dem Gerät befindet sich auf der vom Probanden abgewandten Seite eine Lichtquelle, die einen Lichtstrahl längs einer optischen Achse ausstrahlt. Zwischen der Lichtquelle und dem Auge des Probanden befinden sich zwei Gitter mit zentralen Marken. Der Proband bewegt das Gerät nun so lange, bis sich der Lichtstrahl und die beiden Marken decken. Aus den dabei ermittelten Positionsdaten für das Gerät und den Kopf wird die Lage der Blickachse im Raum errechnet. Der Vorgang wird dann mehrfach unter unterschiedlichen Blickrichtungen wiederholt, so dass mehrere Blickachsen bestimmt werden. Deren Drehpunkt wird dann als Augendrehpunktlage berechnet.

Diese bekannte Vorgehensweise hat den Nachteil, dass ein erheblicher Aufwand an separaten Geräten betrieben werden muss. Ferner ist die Messgenauigkeit vom subjektiven Verhalten des Probanden abhängig.

Aus der EP 0 825 826 A1 sind ein Verfahren und eine Vorrichtung zum parallelen Erfassen von Sehinformationen bekannt. Zum Bestimmen der Augendrehpunktlage wird bei dieser bekannten Vorgehensweise bei fester Ausrichtung und Lage des Kopfes des Probanden mindestens für zwei bekannte, nacheinander von den Augen fixierte Fixierpunkte eine Fixierlinien-Bestimmung durchgeführt. Die Fixierpunkte sind zwei fest mit der Halterung von Kameras und Sensoren zur Bestimmung der Pupillenlage verbindbare Markierpunkte. Der Augendrehpunkt liegt dann im Schnittpunkt der durch die Fixierpunkte gelegten Fixierlinien. Die EP 1 364612 A1 beschreibt eine Augencharakteristik-Messvorrichtung, die eine optische Charakteristik eines Auges misst, und diese mit einem festgelegten Koordinatensystem eines Patientenauges, einer Messvorrichtung oder einer chirurgischen Vorrichtung korreliert.

Die EP 1 767 174 A2 offenbart, ein Operationssystem vor einem chirurgischen Eingriff basierend auf einem bei der Diagnose aufgenommenen Iris-Bild auszurichten.

Der Erfindung liegt die Aufgabe zugrunde ein Verfahren bereit zu stellen, das es möglich macht, beim Erfassen von Parametern des Systems Auge/Brille mit mehreren verschiedenen Messungen unter Verwendung von unterschiedlichen Geräten, eine gemeinsame räumliche Referenz für die Messwerte zu finden.

Diverse im Weiteren verwendete Begriffe sind wie folgt definiert:Die Augenlänge ist die geometrische Länge des Auges zwischen Hornhautscheitel und der Fovea. Unter Augendrehpunktlage versteht man ganz allgemein den Ort des optischen Augendrehpunkts. Als optischer Augendrehpunkt (Kurzzeichen Z') wird z.B. nach der DIN 5340-43 der Fußpunkt des Lotes vom mechanischen Augendrehpunkt auf die ins Augeninnere verlängerte Fixierlinie beim Blick geradeaus auf einen unendlich fernen Punkt bei ungezwungener Kopf- und Körperhaltung angenommen. Der mechanische Augendrehpunkt (Kurzzeichen M) ist z.B. nach der DIN 5340 - 42 derjenige Punkt im Auge, der sich bei Blickbewegungen am wenigsten verlagert.

Bei dem erfindungsgemäßen Verfahren werden bei den photographischen Aufnahmen jeweils zugleich die Referenzstruktur des Auges aufgenommen und die Werte der Parameter auf die Referenzstruktur bezogen.

Das erfindungsgemäße Verfahren umfasst die folgenden Schritte:
a) Messen einer Referenzstruktur des Auges in einer ersten Messsituation;
b) Messen der Referenzstruktur des Auges in einer zweiten Messsituation;
c) Ermitteln der Lageänderung der Referenzstruktur zwischen den beiden Messsituationen;
d) Korrigieren von verordneten Brillengläsern in Abhängigkeit von der Lageänderung.

Dieses Verfahren zeichnet sich dadurch aus, dass man die Messung nach a) und b) aufeinander referenzieren kann.

Durch das Verfahren wird eine bessere Referenzierung zweier mit unterschiedlichen Geräten aufgenommener Parameter möglich und damit Fehlanpassungen einer Brille vermieden.

Schließlich ist eine erfindungsgemäße Vorrichtung zum Bestimmen von mindestens zwei Parametern eines eine Referenzstruktur aufweisenden Auges, mit separaten Aufnahmegeräten zum photographischen Aufnehmen des Auges für die Bestimmung jeweils eines der Parameter das Auge vorgesehen. Die Aufnahmegeräte sind derart ausgebildet, dass bei den photographischen Aufnahmen jeweils zugleich die Referenzstruktur des Auges aufgenommen wird. Es ist (zumindest) eine Recheneinrichtung vorgesehen, um die Werte der Parameter auf die Referenzstruktur zu beziehen. Die obigen Verfahrensschritte a) und b) werden von den beiden Aufnahmeeinrichtungen durchgeführt. Die Verfahrensschritte c) und d) werden von der Recheneinrichtung (z. B. ein Personalcomputer) ausgeführt.

Die der Erfindung zugrunde liegende Aufgabe wird auf diese Weise vollkommen gelöst.

Bei Weiterbildungen dieses Verfahrens wird zum Messen der Referenzstruktur z.B. die Lage der Pupillenmitte des Auges und/oder die Lage des Hornhautscheitels ermittelt. Die Lage der Pupillenmitte oder die Lage des Hornhautscheitels eignen sich besonders als Referenzstrukturen, weil sie einfach zu erfassen sind.

Als Referenzstrukturen eignen sich z.B. auch eine Struktur der Iris oder Blutgefässe der Lederhaut. Derartige Referenzstrukturen sind im Allgemeinen ohne jegliche Symmetrie. Sie erlauben daher eine eindeutige Lokalisierung im Raum.

Ein Computerprogramm mit Programmcode kann zur Durchführung des vorstehend beschriebenen Verfahrens eingerichtet sein, wenn das Programm in einem Computer ausgeführt wird. Das Computerprogramm kann z.B. auf einem maschinenlesbaren Datenträger gespeichert sein.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Figur 1: eine erste schematische Seitenansicht eines nach oben verdrehten Auges zur Erläuterung von verschiedenen Blickrichtungen und Drehpunkten und der Lage des Augendrehpunktes;
- Figur 2: eine zweite schematische Seitenansicht eines geradeaus gerichteten Auges mit vorgesetztem Brillenglas zur Erläuterung weiterer Parameter des Auges;
- Figur 3: eine Frontansicht eines Auges zur Erläuterung bestimmter Augenbereiche;
- Figur 4: eine Vorrichtung zum Bestimmen von mindestens zwei Parametern eines Systems Auge/Brille nach der Erfindung.

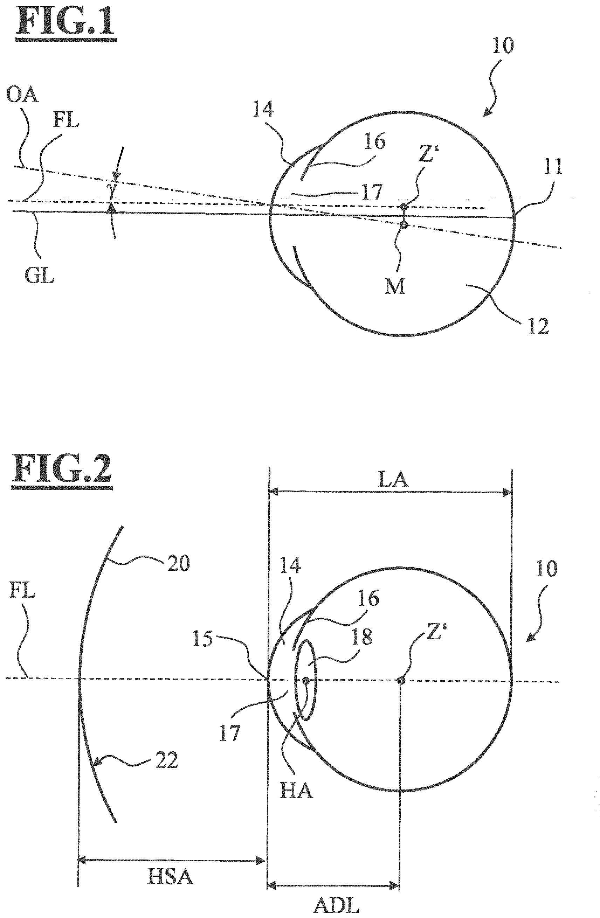

In den Figuren 1 und 2 bezeichnet 10 ein Auge. Das Auge 10 hat einen Glaskörper 12, eine Hornhaut 14, eine Iris 16, eine Pupille 17 sowie eine Linse 18.

Wenn das Auge 10 eine Drehbewegung ausführt, dann geschieht dies nicht exakt um einen Drehpunkt im Raum. Vielmehr gibt es nur einen näherungsweise kugelförmigen Bereich, in dem sich die momentanen Drehpunkte befinden. Derjenige Punkt, der bei Augenbewegungen die geringste Lageveränderung erfährt, wird als mechanischer Augendrehpunkt M bezeichnet (vgl. DIN 5340 - 42).

Mit GL ist die Sehachse (Gesichtslinie) bezeichnet. Sie ist nach DIN 5340 - 360 die Verbindungsgerade zwischen einem fixierten Objektpunkt und dem dazu konjugierten Bildpunkt in der Mitte der Netzhautgrube 11.

Mit FL ist die Fixierlinie (Visierlinie) bezeichnet. Sie ist nach DIN 5340 - 159 die Verbindungsgerade zwischen dem zentral abgebildeten Objektpunkt und der Mitte der Eintrittspupille 17.

OA bezeichnet die optische Achse.

Mit Z' ist der optische Augendrehpunkt bezeichnet. Er ist nach DIN 5340 - 43 der Fußpunkt des Lotes vom mechanischen Augendrehpunkt M auf die Fixierlinie FL.

Der Winkel zwischen der optischen Achse OA und der Fixierlinie FL ist in Figur 1 mit γ bezeichnet. Der Winkel γ ist hier nur in einer Ebene eingezeichnet, symbolisiert aber einen Raumwinkel oben/unten und /rechts/links.

In Figur 2 ist ein Brillenglas 20 vor dem Auge 10 angeordnet. Das Brillenglas hat auf der dem Auge 10 zugewandten Seite eine Rückfläche 22. Der Abstand der Rückfläche 22 von dem Hornhaut-Apex 15 gemessen in Blickrichtung senkrecht zur Fassungsebene wird als Hornhaut-Scheitelabstand HSA bezeichnet (vgl. DIN EN ISO 13666 - 5.27). Der Abstand des Hornhautscheitels 15 vom optischen Augendrehpunkt Z' gibt die Augendrehpunktlage ADL in Bezug auf den Hornhaut-Apex 15 an.

Die Augendrehpunktlage ADL ist ein wichtiger Parameter in der Berechnung des Brillenglases 20. Das Brillenglas 20 wird immer so optimiert, dass es für jede Blickrichtung des Auges 10 die optimalen Abbildungseigenschaften aufweist.

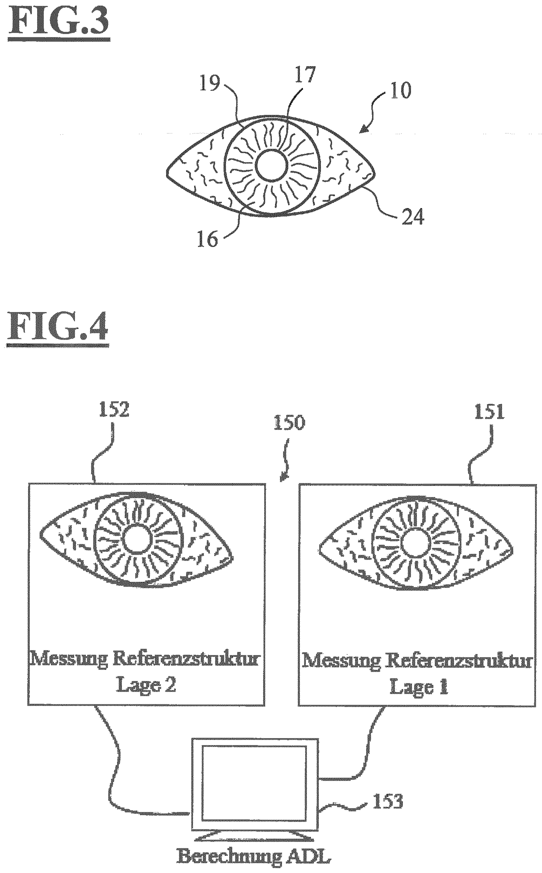

Figur 3 zeigt eine Frontansicht des Auges 10. Man erkennt in der Iris 17 eine charakteristische Struktur sowie neben der Iris 17 in der Lederhaut 24 eine Struktur von kleinen Blutgefäßen.

### Ausführungsbeispiel:

In einem Ausführungsbeispiel eines erfindungsgemäßen Verfahrens wird allgemein in einem ersten Schritt 4a) in einer ersten Messsituation eine Referenzstruktur des Auges 10 erfasst. Die entsprechende Anordnung 150 ist in der Figur 14 dargestellt. Die Referenzstruktur ist dabei vorzugsweise eine Struktur der Iris 16 oder eine Struktur von Blutgefäßen in der Lederhaut 24 (vgl. Bezugszeichen 151).

In einem zweiten Schritt 4b) wird die Referenzstruktur des Auges 10 in einer zweiten Messsituation erfasst. Unter der ersten und der zweiten Messsituation ist zu verstehen, dass zwei unterschiedliche Messungen vorgenommen und/oder Messverfahren eingesetzt wurden, unterschiedlicher Messgeräte 151, 152.

In einem dritten Schritt 4c) wird die Lageveränderung, insbesondere die Verdrehung zwischen den Messsituationen in Schritt 4a) und 4b) rechnerisch (Computer 153) ermittelt und bei der Verordnung des Brillenglases 20 berücksichtigt.

Um in den Schritten 4a) bis 4c) ein gemeinsames Bezugssystem für die gemessenen Lagen der Referenzstrukturen herzustellen, wird erfindungsgemäß in jedem der Schritte 4a) und 4b) eine photographische Aufnahme der Referenzstrukturen hergestellt werden. Diese Referenzstrukturen werden dann als gemeinsames Referenzsystem für die Lagen der Pupillenmitte und des Hornhautscheitels sowie weiterer Parameter verwendet

Dieses Verfahren eignet sich z.B. zur Referenzierung der beiden Aufnahmen des Auges bei unterschiedlichen Blickrichtungen, wie diese beim zweiten und dritten Verfahren erforderlich sind.

### Bezugszeichenliste

- 10: Auge
- 11: Netzhautgrube
- 12: Glaskörper
- 13: Netzhautebene
- 14: Hornhaut
- 15: Hornhautscheitel
- 16: Iris
- 17: Pupille
- 18: Linse
- 19: Limbus
- 20: Brillenglas
- 22: Rückfläche
- 24: Lederhaut
- 150: Vorrichtung zur Bestimmung von mindestens 2 optischen Parametern
- 151: Videozentriergerät (Aufnahme 1)
- 152: Videozentriergerät (Aufnahme 2)
- 153: Computer
- ADL: Augendrehpunktlage
- HA: objektseitiger Hauptpunkt des Auges
- HSA: Hornhaut-Scheitelabstand
- FL: Fixierlinie
- GL: Sehachse
- LA: Augenlänge
- M: mechanischer Augendrehpunkt
- OA: optische Achse
- Z': optischer Augendrehpunkt
- x, y, z: Raumkoordinaten

- γ: Winkel, Blickrichtung

## Patentansprüche

1. Verfahren zum Verordnen eines Brillenglases, bei dem mindestens zwei Parameter eines Systems Auge (10)/Brille bestimmt werden, bei dem für die Bestimmung des jeweiligen Parameters das Auge (10) jeweils mittels eines separaten Aufnahmegerätes photographisch aufgenommen wird, wobei das Auge (10) eine Referenzstruktur aufweist, wobei bei den photographischen Aufnahmen jeweils zugleich die Referenzstruktur des Auges (10) aufgenommen wird und die Werte der Parameter auf die Referenzstruktur bezogen werden, umfassend
a) Messen der Referenzstruktur des Auges (10) in einer ersten Messsituation;
b) Messen der Referenzstruktur des Auges (10) in einer zweiten Messsituation;
c) Ermitteln der Lageänderung der Referenzstruktur zwischen den beiden Messsituationen;
d) Verordnen eines Brillenglases unter Berücksichtigung der ermittelten Lageänderung der Referenzstruktur.

2. Verfahren nach Anspruch 1, **dadurch gekennzeichnet, dass** die Lage der Pupillenmitte des Auges (10) gemessen wird.

3. Verfahren nach Anspruch 1 oder 2, **dadurch gekennzeichnet, dass** zugleich die Lage des Hornhautscheitels ermittelt wird.

4. Verfahren nach einem oder mehreren der Ansprüche 1 bis 3 , **dadurch gekennzeichnet, dass** die Referenzstruktur eine Struktur der Iris (16) und/oder Blutgefäße der Lederhaut (24) sind.

5. Computerprogramm mit Programmcode eingerichtet zur Durchführung des Verfahrens nach einem der vorangegangenen Ansprüche, wenn das Programm in einem Computer ausgeführt wird, welcher eine Vorrichtung gemäss Anspruch 7 steuert.

6. Computerprogramm nach Anspruch 5, gespeichert auf einem maschinenlesbaren Datenträger.

7. Vorrichtung (150), welche eingerichtet ist mindestens zwei Parameter eines Systems Auge (10)/Brille zu bestimmen, mit separaten Aufnahmegeräten (151, 152) zum photographischen Aufnehmen des Auges (10) für die Bestimmung jeweils eines der Parameter, wobei das Auge (10) eine Referenzstruktur aufweist, wobei die Aufnahmegeräte (151, 152) ausgebildet sind, bei den photographischen Aufnahmen jeweils zugleich die Referenzstruktur des Auges (10) aufzunehmen und eine Recheneinrichtung (153) vorgesehen ist, um die Werte der Parameter auf die Referenzstruktur zu beziehen, wobei
a) ein erstes der Aufnahmegeräte (151) eingerichtet ist, die Referenzstruktur des Auges (10) in einer ersten Messsituation zu messen
b) ein zweites der Aufnahmegeräte (152) eingerichtet ist, die Referenzstruktur des Auges (10) in einer zweiten Messsituation zu messen
c) die Recheneinrichtung (153) eingerichtet ist, die Lageänderung der Referenzstruktur zwischen den beiden Messsituationen zu ermitteln und
d) die Recheneinrichtung (153) ferner eingerichtet ist, ein verordnetes Brillenglas in Abhängigkeit von der ermittelten Lageänderung zu korrigieren.

## Claims

1. Method for prescribing a spectacle lens, in which at least two parameters of an eye (10)/spectacles system are determined, in which the eye (10) is photographically recorded, in each case by means of a separate recording device, for determining the respective parameter, wherein the eye (10) has a reference structure, wherein the reference structure of the eye (10) is in each case recorded simultaneously in the photographic recordings and the values of the parameters are related to the reference structure, said method including:
a) measuring the reference structure of the eye (10) in a first measurement situation;
b) measuring the reference structure of the eye (10) in a second measurement situation;
c) ascertaining the change in relative position of the reference structure between the two measurement situations;
d) prescribing a spectacle lens taking account of the ascertained relative change in position of the reference structure.

2. Method according to Claim 1, **characterized in that** the relative position of the pupil centre of the eye (10) is measured.

3. Method according to Claim 1 or 2, **characterized in that** the relative position of the corneal apex is ascertained at the same time.

4. Method according to one or more of Claims 1 to 3, **characterized in that** the reference structure is a structure of the iris (16) and/or blood vessels in the sclera (24).

5. Computer program with program code, configured to carry out the method according to any one of the preceding claims, when the program is executed on a computer which controls an apparatus according to Claim 7.

6. Computer program according to Claim 5, stored on a machine-readable data medium.

7. Apparatus (150), which is configured to determine at least two parameters of an eye (10)/spectacles system, comprising separate recording devices (151, 152) for photographically recording the eye (10) for determining respectively one of the parameters, wherein the eye (10) has a reference structure, wherein the recording devices (151, 152) are embodied to respectively simultaneously record the reference structure of the eye (10) in the photographic recordings and a computing appliance (153) is provided to relate the values of the parameters to the reference structure, wherein
a) a first of the recording devices (151) is configured to measure the reference structure of the eye (10) in a first measurement situation;
b) a second of the recording devices (152) is configured to measure the reference structure of the eye (10) in a second measurement situation;
c) the computing appliance (153) is configured to ascertain the change in relative position of the reference structure between the two measurement situations; and
d) the computing appliance (153) is further configured to correct a prescribed spectacle lens on the basis of the ascertained relative change in position.

## Revendications

1. Procédé de prescription d'un verre de lunettes, dans lequel au moins deux paramètres d'un système œil (10)/lunettes sont déterminés, dans lequel, pour déterminer le paramètre respectif, l'œil (10) est respectivement acquis au moyen d'un dispositif d'acquisition séparé, dans lequel l'œil (10) présente une structure de référence, dans lequel, lors des acquisitions photographiques respectives, la structure de référence de l'œil (10) est acquise simultanément et les valeurs des paramètres sont mises en relation avec la structure de référence, comprenant
a) la mesure de la structure de référence de l'œil (10) dans une première situation de mesure ;
b) la mesure de la structure de référence de l'œil (10) dans une deuxième situation de mesure ;
c) la détermination de la variation de position de la structure de référence entre les deux situations de mesure ;
d) la prescription d'un verre de lunettes en tenant compte de la variation de position déterminée de la structure de référence.

2. Procédé selon la revendication 1, **caractérisé en ce que** la position du centre de la pupille de l'œil (10) est mesurée.

3. Procédé selon la revendication 1 ou 2, **caractérisé en ce que** la position du vertex cornéen est déterminée simultanément.

4. Procédé selon une ou plusieurs des revendications 1 à 3, **caractérisé en ce que** la structure de référence est une structure de l'iris (16) et/ou de vaisseaux sanguins de la sclérotique (24).

5. Programme informatique comportant un code de programme conçu pour mettre en œuvre le procédé selon l'une des revendications précédentes lorsque le programme est exécuté dans un ordinateur qui commande un appareil selon la revendication 7.

6. Programme informatique selon la revendication 5, stocké sur un support de données lisible par machine.

7. Dispositif (150) qui est conçu pour déterminer au moins deux paramètres d'un système œil (10)/lunettes, comportant des appareils d'acquisition séparés (151, 152) permettant l'acquisition photographique de l'œil (10) afin de déterminer respectivement l'un des paramètres, dans lequel l'œil (10) présente une structure de référence, dans lequel les appareils d'acquisition (151, 152) sont conçus, lors des acquisitions photographiques respectives, pour acquérir simultanément la structure de référence de l'œil (10) et un dispositif de calcul (153) est prévu pour mettre en relation les valeurs des paramètres avec la structure de référence,
dans lequel
a) un premier des appareils d'acquisition (151) est conçu pour mesurer la structure de référence de l'œil (10) dans une première situation de mesure
b) un deuxième des appareils d'acquisition (152) est conçu pour mesurer la structure de référence de l'œil (10) dans une deuxième situation de mesure
c) le dispositif de calcul (153) est conçu pour déterminer la variation de position de la structure de référence entre les deux situations de mesure et
d) le dispositif de calcul (153) est en outre conçu pour corriger un verre de lunettes prescrit en fonction de la variation de position déterminée.
